Europäisches Patentamt

European Patent Office

Office européen des brevets

(19)

(11) Publication number: **0 173 092**
**A2**

(12)

# EUROPEAN PATENT APPLICATION

(21) Application number: **85109500.0**

(22) Date of filing: **29.07.85**

(51) Int. Cl.⁴: **A 61 K 37/02**

---

(30) Priority: **03.08.84 US 637421**

(43) Date of publication of application: **05.03.86**
**Bulletin 86/10**

(84) Designated Contracting States: **AT BE CH DE FR GB IT LI LU NL SE**

(71) Applicant: **MEDISEARCH S.A., General Guisan Quay 20, CH-8002 Zurich (CH)**

(72) Inventor: **Bliah, Madeline Andree Myriam, 75016 Rue De La Tour, Paris (FR)**

(74) Representative: **Baillie, Iain Cameron, c/o Ladas & Parry Isartorplatz 5, D-8000 München 2 (DE)**

---

(54) **Anti viral composition.**

(57) Pharmaceutical compositions comprising non-toxic lectins, for example Sambucus nigra agglutinin I are of use in combating viral infections in animals and humans. The amino acid composition of Sambucus nigra agglutinin I and II are given.

EP 0 173 092 A2

-1-

METHODS AND COMPOSITIONS FOR INHIBITING THE
INFECTIOUS ACTIVITY OF VIRUSES

BACKGROUND OF THE INVENTION

The present invention relates to methods and compositions for inhibiting the infectious activity of viruses by the use of lectins. More specifically, it relates to the use of lectins, including those obtained from Sambucus nigra, Sambucus racemosa and **Sambucus** ebulus for inhibiting the activity of **enveloped** viruses.

Viruses may be classified in a number of different ways. However, one major division is between those viruses wherein the virion comprises an envelope surrounding the nucleocapsid and those which do not. Among the viruses wherein the nucleocapsid is enveloped are: (1) DNA viruses such as Herpesviridae including **herpes** simplex, cytomegalovirus, varicella zoster, Epstein Barr and other viruses affecting horses, chickens and cows; Poxviridiae including smallpox, vaccinia, human monkeypox, sheep, **bird**, swine pox viruses and Iridoviridae; (2) RNA viruses such as Togaviridae including alphaviruses such as Sindbis, equine and encephalitis viruses: flaviviruses such as Yellow fever and dengue viruses; rubiviruses such as rubella and pestiviruses; Arenaviridae, Orthomyxoviridae, including influenza types A, B and C viruses; Paramyxoviridae such as parainfluenza (types 1, 2, 3 and 4); respiratory syncitial, mumps, measles and Newcastle disease viruses; Coronaviridae such as coronavirus, Rhabdoviridae; Retroviridae such as onco-

U.S. 637,421

-2-

virinae, spumavirinae and lentivirinae, Bunyaviridae such as bunyavirus; and certain unclassified viruses such as hepatitis A, hepatitis B, non A non B hepatitis viruses, as well as certain viruses connected with tumor production and disorders such as acquired immune deficiency syndrome.

Enveloped animal viruses are characterized by the presence of a membrane consisting of a lipid bilayer with glycoprotein projections, or spikes, on the outer surface of the viral envelope. These spikes consist of virus-coded glycoprotein molecules which are essential for viral infectivity and replication. The antigenic specificity of the virus is also determined by these molecules.

The viral glycoproteins are involved in the early interactions between the virion and the cell, i.e. adsorption to the cell surface and penetration of the virion into the cell. The exact sequence of events in the penetration of enveloped viruses into cells is still not clear. However, in the case of the paramyxo and myxoviruses, it is clear that fusion of viral and cell membranes is involved in the penetration step and that penetration is mediated by a viral glycoprotein. Specific cleavage of the viral glycoprotein activates the ability of the virus to initiate infection.

Lectins are a group of proteins capable of binding specifically to carbohydrate moieties on various cell surfaces. The interaction of a few lectins with glycoproteins on virus envelope has been investigated. It has been found that Concanavalin A and Ricinus communis agglutinin, especially the former, do interfere with the activity of a number of viruses. The toxicity of Concanavalin A and Ricinus communis agglutinin, however, precludes their clinical use (the latter is the most toxic substances known). Other less toxic lectins have been investigated with mixed results.

The ability of Concanavalin A to interfere with viruses has been described in many articles in the

-3-

literature. For example, Calafat and Hageman in J. Gen. Virol $\underline{14}$ : 103-106 (1972) describe the binding of murine RNA tumor viruses with Concanavalin A. Birdwell and Strauss in J. Virol. $\underline{11}$: 502-507 (1973) reported that Concanavalin A and Ricinus communis agglutinin agglutinate Sinbis virus. Stewart et al in Proc. Nat. Acad. Sci. USA $\underline{70}$: 1308-1312 (1973) describe the use of Concanavalin A to selectively agglutinate urine and avian oncornavirions, especially Priend virus. Okado and Kim in Virol. $\underline{50}$: 507-515 (72I showed that the activity of the enveloped viruses of Sendai virus and herpes simplex virus was destroyed by Concanavalin A, but that this lectin had no effect on unenveloped polio virus.

Ito and Barron in J. Virol. $\underline{13}$: 1312-1318 (1974) report that whereas Concanavalin A inhibited the infectivity of herpes simplex virus type 1, phytohaemaglutinin-P. wheat germ agglutinin and pokeweed mitogen had no such effect. They also reported that Concanavalin A inactivated herpes simplex virus type 2, pseudorabies virus and vesicular stomatitis virus, but that there was no such effect on vaccinia, simian andenvirus SV 15 and echovirus type 6. The data suggested that Concanavalin A blocked the binding sites on the virion envelope.

Ito and Barron in J. Gen. Virol. $\underline{33}$: 259-266 (1976) showed that while phytohaemagglutinin-P failed to inactivate herpes simplex virus, it did enhance the inactivation effected by Concanavalin A.

Finkelstein and McWilliams in Virol. $\underline{69}$: 570-586 (1976) reported the effects of a variety of lectins on various viruses. In particular, they reported that phytohaemagglutinin-P, Concanavalin A, Ricinus communis agglutinin and wheat germ agglutinin were effective in inhibiting the growth of Sindbis virus and vaccinia virus in chick embryo and eero cells. They also reported that soybean agglutinin and pokeweed

-4-

mitogen had little effect on the viruses.

Stitz et al in J. Gen. Virol. 34: 523-530 (1977) reported studies of the effect of Concanavalin A on the final stages of replication of fowl plague virus and Newcastle disease virus.

They suggested that a lattice was formed comprising virus particles and Concanavalin A molecules which prevented release of virions.

Cartwright in J. Gen. Virol. 34: 249-256 (1977) concluded that the effect of Concanavalin A in preventing the replication of mature esicclar stomatitis virus was due to blockage of the glycoprotein receptor sites on the cell by the lectin.

Ito et al in Arch. Virol. 57: 97-105 (1978) report that human cytomegalovirus (a herpesvirus) can be inactivated by phytohaemagglutinin whereas herpes simplex virus is not and that wheat germ agglutinin and pokeweed mitogen were ineffective against both viruses.

Urade et al in the Arch. Virol. 56: 35936 (1978) reported that the infectivity of wild type rubella viruses was destroyed by Concanavalin A whereas this lectin had little effect on rubella pi viriants. The authors concluded that the difference resulted from a difference in carbohydrate content in the structure of the envelopes.

Delaneau et al reported in Ann. Virol. (Inst. Posteur) 132 E.: 461-471 (1981) that certain lectins, phytohaemagglutinin-Els, wheat germ agglutinin, Ricinus communis agglutinin, Lens culinaris agglutinin and to a lesser extent Concanavalin A, form aggregates in vitro with rabies virus, whereas limulin had no such effect. Arachis hypogalectin was only effective in causing agglutination if the virus particles were desalinated.

Ziegler and Pozos reported in Infect. Immunity 34: 588-595 (1981) that Concanavalin A binds to the herpes simplex virus and renders it inactive. A similar report with respect to succinyl Concanavalin A was published by Garrity et al in Antimicrob. Agents Chemotherapy 21: 450-55 (1982).

Olofsson et al in Arch. Virol. 76: 258 8 (1983) suggested that the affinity of herpes simplex virus glycoprotein for Helix pomatia lectin was due to the presence of N-acetyl galactosamine as terminal sugar in the oligosaccharide of the virion envelope.

Klein's U.S. Patent 4,197,294 disclosed the feeding of vegetable materials, including elderberries and elderflowers of an unspecified species to chicken to increase the iodine, niacin hormones, calcium and magnesium in their diets. There is no disclosure of the presence on use of a lectin.

Sugimoto's U.S. Patent 4,296,025 disclosed the use of a toxic lectin (phytohaemagglutinin) in the laboratory production of interferon.

Yoshii Chemical Abstracts Vol. 49 8402 (e) discusses the reaction of leaf press juices of various plants on a nonenveloped virus (tobacco mosaic virus). Juice from inter alia Sambacus siebholdiana was found to have a powerful inhibitious effect on tobacco mosaic virus infection.

Furassawa in Chemical Abstracts Vol. 74 86207k disclosed the activity of alkaloids derived from bulbs of Narcissus tazetta and from Sambucus siebholdiana against certain viruses. The present invention uses lectins which are glycoproteins, not alkaloids. Alkaloids are generally toxic and not suitable for pharmaceutical use. Those of sambucus plants are found in inedible parts of the plant.

In the present inventors PLD thesis, submitted to the Ecole Polytechnique Federade at Zurich and which became available to the public in the Swiss National Library on September 1, 1983, there are described in vitro studies of the effect of Sambucus nigra agglutinin I and II on the agglutinaton of animal (including human) erythrocytes and lymphocyles, or the agglutination

-6-

of certain animal erythromytes in the absence and also the presence of fowl pest virus and on the abstracts of these agglutinin to inhibit the infections of fowl pet virus of Cancerous human kidney cells.

Without wishing to be bound by any theory, we believe that the lectins bind themselves to the virions thereby resulting in agglutination of virus articles which prevent their penetration to cells. It is, however, possible that alternative mechanisms are involved, for example, that the lectins bind to the cell surface thereby blocking virus receptor sites on the cell wall, or that by modifying the surface of the cell wall the lectins act to lock virus into the cell membrane, thereby preventing the release of viral replicates. It is even possible that lectins act in same way to interfere with the intracellular replication of viruses.

The applicant belives it possible that the mode of action of certain traditional herbal or fruit-based remedies from various disorders such as the common cold may have been through lectins.

One purpose of the present invention is to use non-toxic lectins for prevention or treatment of diseases caused by enveloped viruses.

Another purpose of the invention is to provide a pharmaceutical basis and mechanism for the anti activity.

Pharmaceutical regimen plant extracts containing some of the plants from which the lectins to be used in the present invention are derived have previously found medical use. For example, an extract of Sambucus nigra has been used as a diaphoretic, a diuretic and a cathartic agent; Datura stramonium has been used as agent against coughs and laryngitis; Phytolacca has been used as an antirheumatic preparation and as a topical antiparasitic agent. Solanm tuberosum has been used as a cpasmolytic agent. We have further identified the composition of the active components of Sambucus nigra $\underline{1}$ agglutinin.

-7-

## SUMMARY OF THE INVENTION

Accordingly, in the first place, the present invention provides pharmaceutical composition comprising a virus inhibiting concentration of a non-toxic lectin and a pharmaceutically acceptable carrier, diluent, encapsulating agent or solvent.

From a further aspect, the invention provides an anti-viral composition comprising a lectin containing as grams per 100 grams of polypeptide:

| Aspartic acid | about | 16.3 |
|---|---|---|
| Threonine | about | 6.8 |
| Serine | about | 7.0 |
| Glutamic acid | about | 11.8 |
| Proline | about | 3.8 |
| Glycine | about | 4.4 |
| Alanine | about | 3.1 |
| Half-cystine | about | 2.9 |
| Valine | about | 6.0 |
| Methionine | about | 3.1 |
| Isoleucine | about | 8.0 |
| Leucine | about | 7.7 |
| Tyrosine | about | 2.7 |
| Phenylalanine | about | 3.3 |
| Histidine | about | 0.4 |
| Lysine | about | 1.8 |
| Arginine | about | 5.4 |

## DETAILED DESCRIPTION OF THE INVENTION

Non-toxic lectins for use in the composition and regimens proposed in the present invention are those lectins which are non-toxic ($LD_{50}$ 50% effective dose) when administered intravenously to experimental animals at doses required for effective inhibition of viral infections. Such lectins, to be used in the proposed forms and dosages, include Sambucus nigra agglutinin I and II, Sambucus racemosa, Sambucus ebulus, Agaricus bisporous, Anguilla anguilla, Arachis hypogaea, Bandeiraea

-8-

simplicifalia I, II, Bauhinia purpurea, Cytisus sessilifolius, Datura stamonium, Ertythrina cristogalli, Euonymus europeus, Helix aspersa, Helix pamatia, Iberis amara, Laburnum alpinum, Lens culinaris, Limax falvus, Limulus polyphemus, Lotus tetragonolubus, Maclura pomifera, Mangifera indica, Perseau americana, Phytolacca americana, Pisum sativum, Robinia pseudoacacia, Salvia horminum, Salvia scalrea, Sarothamnus scoparius, Solanum tuberosum, Sophora japonica, Trichosanthes kinlowii, Triticum vulgaris, Ulex europaeus I, II, Vicia faba, Vicia graminea, Vicia villosa, Wisteria floribundo, Codium fragile, Lycopersicon esculentum, Mycoplasma gallisepticum, Pelvetia canaliculata, Ptilota plumosa, Tetragonolobus purpureas (asparagus pea, winged pea, Lotus tetragonolobus).

With the exception of the Sambucus lectins, all these lectins are commercially available for research purposes from Medac Laboratory of Hamburg, West Germany and/or from Sigma Chemicals of St. Louis, USA.

Lectins which are not commercially available may be obtained by standard extractive procedures. Lectins can be purified from saline extracts of plants by conventional techniques for protein purification such as salt precipitation, gel chromatography, ion exchange chromatography, preparative electrophresis and related techniques. Based on the ability of lectins to specifically and reversibly bind saccharides, the affinity chromatography can be used for lectin purification, since the experimental conditions are mild and cause only little, if any, damage to the lectin. For example, Sambucus nigra agglutinins I and II may be obtained from elderberries by affinity chromatography on Sepharose-galactose column.

For example, elderberries from Sambucus nigra I may be pressed without crushing the seeds and the extract recovered by centrifugation and filtration. The extract should then be ultra-centrifuged. The lectins may be recovered from the extract by affinity chromatography on a sepharose-galactose column followed by election with lactose solution (e.g. 0.2M lactose solution). The lactose may be removed (for example, by passage through a Sephadex G25 column). The desorped material is then resubjected to affinity chromatography on a sepharose-galactose column. The first two peaks recovered during desorption are dialyzed against water and lyophilized. The first peak comprises Sambucus nigra II lectin which is not appreciably adsorbed on to the sepharose-galactose column and the second peak comprises Sambucus nigra 1 lectin.

Sambucus nigra agglutinin I is a monomeric glycoprotein with an apparent molecular weight of 40,000 and consists of four isoforms with isoelectric points in the interval 4.25-4.55. It agglutinates untreated human erythrocytes of all blood groups as well as rabbit, chicken and guinea-pig blood cells, the agglutination is inhibited by D-galactose and various galactosides. The best inhibitors are N-acetylgalactosamine and thiodigalactoside.

Sambucus nigra agglutinin II has a molecular weight of 83,000 in its native form and about 40,000 when reduced. It is inhibited by various D galactosides. The best inhibitor is N-acetyl galactosamine. The affinity of Sambucus nigra agglutinin I for galactose is about 40 times higher than the affinity of Sambucus nigra agglutinin II for this sugar and therefore the agglutinins can be separated by affinity on a galactose-Sepharose column.

It was determined that the composition of the (Sambucus nigra agglutinin I lectin) had the following amino acid composition in grams per 100 graspolypepticle:

| | |
|---|---|
| Aspartic acid | 16.3 |
| Threonine | 6.8 |
| Serine | 7.0 |
| Glutamic acid | 11.8 |
| Proline | 3.8 |

-10-

| | |
|---|---|
| Glycine | 4.5 |
| Alanine | 3.1 |
| Half-cystine | 2.9 |
| Valine | 6.0 |
| Methionine | 3.1 |
| Isoleucine | 8.0 |
| Leucine | 7.7 |
| Tyrosine | 2.7 |
| Phenylalanine | 3.3 |
| Histidine | 0.4 |
| Lysine | 1.8 |
| Arginine | 5.4 |

The composition of Sambucus nigra agglutinin II had the following amino acid composition per 100 grams polypepticle:

| | |
|---|---|
| Aspartic acid | 12.6 |
| Threonine | 7.9 |
| Serine | 6.9 |
| Glutamic acid | 12.0 |
| Proline | 4.4 |
| Glycine | 4.1 |
| Alanine | 3.5 |
| Half-cystine | 2.2 |
| Valine | 9.1 |
| Methionine | 1.6 |
| Isoleucine | 5.4 |
| Leucine | 10.1 |
| Tyrosine | 4.7 |
| Phenylalanine | 4.7 |
| Histidine | 1.0 |
| Lysine | 2.5 |
| Arginine | 7.6 |
| Tryptophan | n.d. |

Purifications of the peanut lectin (Arachis hypagaea) and of the soybean lectin were performed by affinity chromatography on Sepharose - - aminocaproyl,

-11-

-D galactopyranosylamine. Solanum tuberosum agglutinin
is extracted from potato tubers by chromatography on
G 100 Sephadex column.

It is not necessary that the lectin by isolated
in absolutely pure state, although this is often desirable
for detailed pharmacokinetic studies.

The formulation and administration of drugs
containing lectins will take into account the inactivation
of the lectin by sugars or carbohydrate containing substances. Preparations with Sambucus nigra I should not
contain galactose, lactose or melibiose and should not
be administered either with dairy products or honey.
During the drug processing the temperature should not
exceed 70°C. since some lectins are destroyed by heat
at that level.

The lectins may be administered in any convenient form, route and mode of entry depending upon
nature of virus to be takled. For example, an ointment
or solution for topical application may be of use in
treating an ocular or genital herpes infection whereas
a nose and throat spray will be of most use in treating
influenza. It will be recalled that many viruses
replicate only in selective tissues.

If the lectins are applied in the vicinity
of the tissue in which replication occurs, there may be
no need for systemic treatment. In the case of influenza
virus, the primary source of infection is the mucosa of
the respiratory tract, hence control of replication of
the virus there can bring fast relief to the patient.
On the other hand, systemic therapy will often be required.
Lectins may be administered in any convenient form. In
many cases, it will be convenient to administer the lectin
orally or parenterally in liquid composition. Formulations
containing 0.2 mg/ml up to 20 mg/ml lectin may be employed.

Typically, the patient will be given a dosage
of 0.02 to 1g daily.

In the case where a liquid formulation is

-12-

intended for topical use, (i.e. in the treatment of herpes infection of the eye) the formulation may have to meet other requirements. For example, a solution for ocular use should be isotonic with eye fluids, nonirritating to the eye and sterile.

Liquid formulations may be prepared for administration in any convenient manner. In the case of formulations for parenteral administration, a solution of the lectin will be supplied in the form of a single or multiple-dose vial or ampule. Vehicles suitable for parenteral injection may be either aqueous or non-aqueous such as fixed oils of vegetable origin (cotton seed, peanut oil, corn, sesame), dimethylsuloxide and other non-aqueous vehicles which will not interfere with therapeutic efficiency of the preparation and are non-toxic in the volume or proportion used.

In the case of solid formulations, tablets are normally formed by mixing the lectin with a binder and a diluent such as lactose or starch. In the case of lectin with affinity for lactose, the use of lactose as adjuvant in the preparation of tablets must be avoided. If the lectin is destroyed or inactivated by the gastric juice or if it may irritate the gastric mucosa, it may be desirable to provide the tablet with an enteric coating. On the other hand, if the purpose of administering the lectin is to deal with viruses present in the gastrointestinal tract such a coating will not be provided.

A lectin-containing tablet will contain 0.02-0.2g of lectin, together with pharmaceutically-acceptable binders or carriers. The concentration of lectin in the tablet will constitute 20-99% of the tablet weight.

Lectins may also be administered as capsules. In this case, the lectin may be combined with a diluent or carrier as described above, but no binder will be used and the mix will not be subjected to a tableting step. Dosage unit amounts of the lectin/carrier mixtures, or, if appropriate, the lectin will be encapsulated in

-13-

gelatin or a similar film-forming encapsulating agent.

It may also be desirable to provide the lectin in a prolonged release formulation wherein a capsule is provided containing a pellet of smaller capsules or particles each of which is capable of being broken down by body fluids at a different rate.

Lectin formulations for topical applications are formulated in a semi-solid material such as oleaginous ointment bases (for example, white petrolatum) oil in water or water in oil emulsions, water removable bases and water-soluble bases.

In the case of a formulation which is intended to be inhaled, it may be desirable to formulate this in a low pressure aerosol can which contains the lectin either in powdered solid form or as a suspension or dispersion. Such formulations also comprise a propellant such as fluorochloro-derivatives of methane and methane.

When used to control viral infections in domestic animals, the lectin may be incorporated into the animals' feed. For example, the lectins may be included in a premix together with various minerals or antibiotics.

In my own investigation of the effects of Sambucus nigra agglutinin I on viral interaction with mammalian cells, I have obtained the following results:

1) Abrogation of cytophathic effect induced by cytomegalovirus on human lung fibroblastic monolayers was effected by Sambucus nigra agglutinin I at a concentration of 100 /ml.

2) Immortalization of human cord blood lymphocytes induced by Epstein-Barr virus was prevented by Sambucus nigra agglutinin I at a concentration of 200 /ml.

3) Cytopathic effect induced by herpes virus type I in human embryonic lung fibroblasts was prevented by Sambucus nigra agglutinin II at a concentration of 200 /ml.

-14-

Based on projected extrapolations, effective doses comparable for an adult should be between 0.6 and 1.2g to reach comparable concentrations of lectin in the blood circulation.

PHARMACOLOGY TESTING

Series 1

Four groups each of 20 male C57 black mice aged 5 to 6 weeks tested. To group 1 there was administered intra-nasally 0.1 ml. of the suspension of virus PR8/34 CH$_{O_{N1})}$ (an influenza virus type A) which had been cultivated in the allantone cavity of 10-11 day old embryonated chicken eggs.

To group II there was administered a similar suspension which had been incubated with 50 Sambucus nigra agglutinin I lectin.

To group III there was administered only the Sambucus nigra agglutinin I lectin.

Group IV was a control group to which no virus or lectin was administered.

In group I, 8 mice died 6 days after administration of the virus, 5 mice died 7 days after administration, 3 mice died after 8 days and the remaining mice died 9 days after the administration.

In group II, one mouse died after 9 days and one mouse died after 11 days. There were no other mortalities in group II.

There was no mortalities in groups III and IV.

Three weeks after administration, the surviving members of group II were sacrificed and examined. It was determined that these mice had contracted influenza but had recovered.

Series 2

IN VITRO ASSAY FOR NEUTRALIZATION OF INFLUENZA VIRUS (FOWL PLAGUE VIRUS) - INFECTIVITY BY SABUCUS NIGRA 1 LECTIN

The assay was carried out in microtiter plates containing human renal carcinoma monolayer cell cultures.

-15-

The monolayers were washed.  The influence of SNA I was determined by 5 minutes incubation of 0.1 ml of two-fold dilutions of the virus with 0.1 ml of a 50 g/ml lectin solution.  The assay was followed visually during the incubation at 37°C. using a phase contrast microscope. The monolayers were checked for cytopathic effect and colored with fuchsin-methylene blue when the virus controls were completely lysed.  Cell controls were performed in the presence and in the absence of lectin. The number of plague forming units in the virus suspension was determined.

The assay showed that **incubation** of the virus with the lectin provides an effective neutralization of the infectivity of the virus in vitro.  The assay was repeated, using different concentrations of lectins and virus and the results indicate that, approximately estimated, one plague forming unit can be neutralized by 0.5 g/ml lectin solution.

Series 3

IN VIVO EXPERIMENTS IN MICE

C 57 -6 weeks old mice were administered intranasally with an influenza virus+ A/PR8/34 (HON1) with or without prior incubation with 5 o g Sambucus nigra lectin II.  After 14 days, all mice infested by the virus died, whereas there was a sufficient reduction (90%) of the mortality in the group which received the virus previously incubated with the lectin.

-1-

## C L A I M S

1.      A pharmaceutical composition consisting of viral replicative inhibiting concentration of a non-toxic lectin and a pharmaceutically-acceptable carrier, diluent encapsulating agent or solvent.

2.      A pharmaceutical composition according to claim 1, which comprises a lectin selected from the group consisting of Sambucus nigra agglutinin I and Sambucus nigra agglutinin II.

3.      A pharmaceutical composition according to claim 2, wherein the composition is liquid emulsion or suspension and comprises from 0.2 to 20 mg/ml of lectin.

4.      A pharmaceutical composition according to claim 3, wherein the composition is in tablet form, each tablet comprising from 0.02 to 0.2g of lectin.

5.      A pharmaceutical composition according to claim 2, wherein the composition is in capsule form, each capsule comprising from 0.02 to 0.2g of lectin.

6.      A pharmaceutical composition according to claim 2, wherein the composition is in the form of an aerosol spray and further comprises a pharmaceutically-acceptable propellant.

7.      A pharmaceutical composition according to claim 2, which is in the form of an ointment or a paste or a solution.

8.      A pharmaceutical composition consisting of a viral replicative inhibiting concentration of a lectin containing per 100 parts by weight of polypeptide the following amino acids in the stated parts by weight:

| | | |
|---|---|---|
| Aspartic acid | about | 16.3 |
| Threonine | about | 6.8 |
| Serine | about | 7.0 |
| Glutamic acid | about | 11.8 |
| Proline | about | 3.8 |
| Glycine | about | 4.5 |
| Alanine | about | 3.1 |
| Half-cystine | about | 2.9 |

U.S. 637,421

-2-

| | | |
|---|---|---|
| Valine | about | 6.0 |
| Methionine | about | 3.1 |
| Isoleucine | about | 8.0 |
| Leucine | about | 7.7 |
| Tyrosine | about | 2.7 |
| Phenylalanine | about | .3 |
| Histidine | about | 0.4 |
| Lysine | about | 1.8 |
| Arginine | about | 5.4 |

and a pharmaceutically-acceptable carrier, diluent, encapsulating agent or solvent.

9. A pharmaceutical composition according to claim 8, wherein said composition is in tablet or capsule form, each tablet or capsule comprising 0.02 to 0.2g of said lectin.

10. A pharmaceutical composition according to claim 8, in the form of an aerosol spray and further comprising a pharmaceutically-acceptable propellant.

11. A pharmaceutical composition according to claim 8, which is in the form of an ointment, paste or solution.

12. Isolated Sambucus nigra agglutinin I for use as a therapeutic agent.